# EUROPÄISCHE PATENTANMELDUNG

(11) **EP 2 301 539 A1**
(43) Veröffentlichungstag der Anmeldung: **30.03.2011**
(21) Anmeldenummer: 09169657.5
(22) Anmeldetag: 07.09.2009
(51) Int. Cl.: A61K 31/426, A61K 31/4164, A61P 37/00

(54) **PPAR-gamma-Agonisten zur Behandlung von Erkrankungen mit pathophysiologischer Beteiligung von TH17-Lymphozyten**

(71) Anmelder: Rheinische Friedrich-Wilhelms Universität, 53115 Bonn (DE)
(72) Erfinder: Knolle, Percy, 81735 München (DE)
(74) Vertreter: Althausen, Sonja

(57) **Zusammenfassung**

Die vorliegende Erfindung betrifft die Verwendung von PPARγ-Agonisten zur Herstellung eines Medikaments zur Behandlung von Erkrankungen, bei denen T_{H}17-Zellen eine pathophysiologisch relevante Funktion haben, sowie diese Medikamente, und erfasst auch Verfahren zur Behandlung von Erkrankungen, bei denen T_{H}17-Zellen eine pathophysiologisch relevante Funktion haben, sowie Verfahren zur Reduktion der Differenzierung von CD4+ T-Lymphozyten zu T_{H}17-Lymphozyten und/oder zur Hemmung der Aktivität und/oder Reduktion der Expression des Transkriptionsfaktors RORγt mittels PPARγ-Agonisten.

## Beschreibung

Die vorliegende Erfindung betrifft die Verwendung von Agonisten des peroxisomalen Proliferator-aktivierten Rezeptors gamma (PPARγ) zur Herstellung eines Medikaments zur Behandlung von Erkrankungen, bei denen T_{H}17-Lymphozyten eine pathophysiologisch relevante Funktion haben.

Die vorliegende Erfindung betrifft auch die Verwendung von Agonisten des peroxisomalen Proliferator-aktivierten Rezeptors gamma (PPARγ) zur Behandlung von Erkrankungen, bei denen T_{H}17-Lymphozyten eine pathophysiologisch relevante Funktion haben.

Die vorliegende Erfindung betrifft ferner ein Verfahren zur Reduktion der Differenzierung von CD4⁺ T-Lymphozyten zu T_{H}17-Lymphozyten durch pharmakologische Aktivierung von PPARγ in CD4⁺ T-Lymphozyten.

Autoimmunerkrankungen entstehen, wenn sich das Immunsystem eines Organismus in unerwünschter Weise gegen normale Gewebe dieses Organismus richtet. CD4⁺ T-Zellvermittelte Autoimmunerkrankungen umfassen eine Reihe chronischer, unheilbarer Erkrankungen, die zu einer zunehmenden Behinderung mit langfristiger Einschränkung von Lebensqualität, Arbeitsfähigkeit und Mobilität führen. In diese Gruppe von Erkrankungen gehören zum Beispiel Multiple Sklerose (MS), rheumatoide Arthritis, Morbus Crohn und Colitis ulcerosa, systemische Vaskulitiden wie Lupus erythematodes und die Psoriasis. Trotz der Vielzahl von Erkrankungen mit ganz unterschiedlichen Zielorganen und dadurch bedingt unterschiedlichen Krankheitsmanifestationen ist als gemeinsame pathophysiologische Grundlage eine pathologische Aktivierung autoreaktiver T-Lymphozyten nachgewiesen worden. Diese Aktivierung führt zur Expansion und Differenzierung der autoreaktiven T-Zellen in verschiedene Effektorzellen. Bis vor wenigen Jahren ging man davon aus, das sich CD4⁺ T-Zellen entweder in IFNγ produzierende TH₁-Zellen oder in IL-4 produzierende T_{H}2-Zellen differenzieren. Insbesondere IFNγ produzierende T_{H}1-Zellen wurden für die Entwicklung CD4⁺-vermittelter Autoimmunität verantwortlich gemacht.

Die therapeutischen Optionen bei den verschiedenen CD4⁺ T-Zell-vermittelten Autoimmunerkrankungen sind darauf ausgerichtet, die pathologische Aktivierung autoreaktiver Zellen zu reduzieren und somit die Krankheitsmanifestation zu vermindern. Die meisten Therapeutika, beispielsweise Cortison oder klassische Immunsuppressiva wie Azathioprin oder Cyclophosphamid, reduzieren unselektiv die Aktivität sämtlicher Immunzellen. Die Therapiestrategien mit diesen Therapeutika sind aufgrund des unselektiven Wirkmechanismus der Therapeutika und der daraus resultierenden Suppression des Immunsystems mit zahlreichen Nebenwirkungen behaftet. Modernere Immunmodulatoren wie Interferon-β, das für eine Therapie bei schubförmiger Multipler Sklerose verwendet wird, oder eine Therapie mit neutralisierende Antikörper gegen TNFα, die zur Therapie rheumatoider Arthritis zum Einsatz kommen, weisen einen selektiveren Wirkmechanismus auf, haben jedoch nur eine eingeschränkte Wirksamkeit. So wird beispielsweise bei einer Interferon-β Therapie bei Multipler Sklerose nur eine Schubratenreduktion von ca. 30% pro Jahr erreicht. Erfolgreiche kurative Therapieansätze existieren bisher nicht.

In den letzten drei Jahren wurde eine neue Gruppe von CD4⁺ Effektor-T-Lymphozyten charakterisiert, nämlich IL-17A produzierende T_{H}17-Lymphozyten. Diese Gruppe stellt neben den TH₁-Zellen und den T_{H}2-Zellen eine eigene unabhängige Effektor-T-Zelllinie dar. Besondere Aufmerksamkeit wurde den T_{H}17-Zellen deshalb zuteil, weil sie mittlerweile für zentrale Effektorzellen in der Pathophysiologie verschiedener Autoimmunerkrankungen gehalten werden.

Insbesondere im Tiermodell der Multiplen Sklerose, der experimentellen autoimmunen Enzephalomyelitis, konnte gezeigt werden, dass die ursprünglich für die Erkrankung verantwortlich gemachten T_{H}1-Zellen keine entscheidende Rolle in der Pathogenese dieser Erkrankung spielen. Die Erkrankung steht eindeutig mit T_{H}17-Zellen in Zusammenhang. Mittlerweile gibt es Evidenzen für eine pathogenetische Rolle von T_{H}17-Zellen in folgenden Erkrankungen: rheumatoide Arthritis, Psoriasis, Multiple Sklerose, chronisch entzündliche Darmerkrankungen wie zum Beispiel Morbus Crohn, Colitis ulcerosa und systemischer Lupus erythematodes (Tesmer, L. A. et al., Immunol. Rev. (2008) 223:87-113; Wong, C. K. et al., Clin. Immunol. (2008) 127:385-393; Fujino, S. et al., Gut (2003) 52:65-70). Insbesondere neue Ergebnisse zeigen, dass T_{H}17-Zellen auch eine zentrale pathogenetische Rolle beim cerebrovaskulären Insult, dem allergischen Asthma bronchiale und dem akuten Koronarsyndrom spielen (Tesmer, L. A. et al., Immunol. Rev. (2008) 223:87-113; Shichita, T. et al., Nat. Med. (2009) 15:946-950; Cheng, X. et al., Clin. Immunol. (2008) 127:89-97).

Die der vorliegenden Erfindung zugrunde liegende Aufgabe bestand darin, Medikamente und Therapiemöglichkeiten zur Behandlung von Erkrankungen bereitzustellen, bei denen T_{H}17-Lymphozyten eine pathophysiologisch relevante Funktion haben.

Die vorliegende Erfindung basiert auf experimentellen Ergebnissen, die zeigen, dass eine Aktivierung von PPARγ in CD4⁺ T-Zellen während der Differenzierung zu T_{H}17-Zellen zu einer ausgeprägten Inhibition der IL-17A Expression führt.

Der peroxisomale Proliferator-aktivierte Rezeptor gamma (PPARγ) ist ein nukleärer Transkriptionsfaktor, der an der Regulierung von Fett- und Glukosestoffwechsel, Zelldifferenzierung sowie inflammatorischen Prozessen beteiligt ist (Daynes, R. A., und Jones, D. C. Nat. Rev. Immunol. (2002) 2:748-759). Der Rezeptor wird unter anderem im Gehirn (Klotz, L. et al., J. Neurochem. (2003) 86:907-916), in T-Lymphozyten und B-Lymphozyten (Clark, R. B. et al., J. Immunol. (2000) 164:1364-1371) und in dendritischen Zellen (Nencioni, A. et al., J. Immunol. (2002) 169:1228-1235) exprimiert. Eine ligandenabhängige Aktivierung ermöglicht die Bindung von PPARγ an eine spezifische Erkennungssequenz (PPRE) im Promotorbereich des jeweiligen Zielgens und dadurch die direkte Modulation der Transkription (Straus, D. S. und Glass, C. K. Trends Immunol. (2007) 28:551-558). Eine Reihe antiinflammatorischer Effekte von PPARγ werden indirekt durch Transrepression vermittelt, also durch hemmende Interaktion mit anderen, proinflammatorischen Transkriptionsfaktoren wie zum Beispiel NFKB (Jiang, Y. et al. Acta Biochim. Biophys. Sin. (Shanghai) (2007) 39:366-376; Ricote, M. et al., Nature (1998) 391:79-82). Zu den bisher bekannten PPARγ-aktivierenden Substanzen gehören zum einen endogene Liganden wie 9s-HODE und 13s-HODE (Huang, J. T. et al. Nature (1999) 400:378-382), zum anderen nichtsteroidale Antiphlogistika und die Gruppe der Thiazolidinedione (Pioglitazone, Actos, Takeda Pharma; und Rosiglitazone, Avandia, GlaxoSmithKline), die als Antidiabetika zur metabolischen Therapie bei Diabetes Mellitus Typ II klinisch angewendet werden. Für diese Medikamente liegt neben der Therapie des Diabetes Mellitus Typ II keine Zulassung für eine weitere Indikation vor.

In den letzten Jahren wurde eine Reihe antiinflammatorischer Effekte von PPARγ-Agonisten auf Zellen des Immunsystems charakterisiert. Neben seit längerem bekannten Effekten auf Antigen präsentierende Zellen des innaten Immunsystems wie Makrophagen und dendritische Zellen konnten in jüngerer Zeit immunmodulierende Effekte auf Zellen des adaptiven Immunsystems charakterisiert werden: Behandlung aktivierter CD4⁺ T-Lymphozyten mit PPARγ-Agonisten führte zur Hemmung der Proliferation von T-Zellen und der IL-2 Sekretion (Clark, R. B. et al., J. Immunol. (2000) 164:1364-1371). Auf B-Lymphozyten wirken PPARγ-Agonisten ebenfalls antiproliferativ (Schlezinger, J. et al., J. Immunol. (2002) 169:6831-6841 ). Trotz der zahlreichen Belege für immunmodulatorische Effekte verschiedener PPARγ-Agonisten konnte bisher nicht geklärt werden, ob die Aktivierung des Rezeptors selbst oder andere, rezeptorunabhängige Mechanismen für die immunmodulatorischen und antiinflammatorischen Effekte dieser Substanzen verantwortlich sind. So wurde zum Beispiel an aus embryonalen Stammzellen generierten PPARγ^{-/-} Makrophagen gezeigt, dass Thiazolidinedione PPARγunabhängig antiinflammatorisch wirken, da die antiinflammatorischen Effekte in PPARγ^{-/-} Makrophagen genauso ausgeprägt waren wie in Wildtyp-Makrophagen (Chawla, A. et al., Nat. Med. (2001) 7:48-52). Darüber hinaus gibt es einige Arbeiten, in denen nicht alle untersuchten PPARγ-Agonisten antiinflammatorische Effekte aufwiesen, oder bei denen die für die antiinflammatorischen Effekte erforderlichen Konzentrationen weit über den für die Rezeptoraktivierung nötigen Konzentrationen lagen (Chawla, A. et al., Nat. Med. (2001) 7:48-52; Rossi, A. et al., Nature (2000) 403:103-108; Ward, C. et al., J. Immunol. (2002) 168:6232-6243). Die Beurteilung des Wirkmechanismus von PPARγ-Agonisten wurde bisher zusätzlich erschwert, da die Generierung einer PPARγ knock-out Maus aufgrund einer durch eine Plazentadysfunktion verursachten embryonalen Letalität gescheitert ist (Barak, Y. et al., Mol. Cell (1999) 4:585-595).

Sowohl im Tiermodell der Multiplen Sklerose als auch bei experimentell induzierter Arthritis und Colitis wurden antientzündliche Effekte von verschiedenen PPARγ-Agonisten nachgewiesen, die zumindest im jeweiligen Tiermodell eine signifikante Verbesserung der klinischen und histopathologischen Krankheitsmanifestationen zur Folge hatten (Saubermann, L. J. et al., Inflamm. Bowel Dis. (2002) 8:330-339; Feinstein, D. L. et al., Ann. Neurol. (2002) 51:694-702; Kobayashi, T. et al., Arthritis Rheum. (2005) 52:479-487). Es ist jedoch nicht bekannt, ob es sich hierbei wirklich um rezeptorspezifische Effekte handelt und welcher Zelltyp für die Vermittlung dieser Effekte verantwortlich ist. Insbesondere ist nicht untersucht, ob ein Effekt auf CD4⁺ T-Lymphozyten an der antientzündlichen Wirkung von PPARγ-Agonisten in den verschiedenen genannten Tiermodellen beteiligt ist. Die Wirkung von PPARγ-Agonisten auf die Differenzierung und Aktivität von T_{H}17-Zellen ist bisher weder in-vitro noch in-vivo untersucht worden.

Mit der Offenlegungsschrift US 2009/0082260 A1 wird ein Verfahren zur Behandlung oder Vermeidung einer unerwünschten Immunantwort beschrieben, bei dem gleichzeitig ein die Immunantwort unterdrückender Wirkstoff wie Cyclosporin und ein PPARγ-Agonist verabreicht werden.

In der Offenlegungsschrift US 2009/0136470 A1 werden Verfahren zur Stimulation der Differenzierung zu regulatorischen T-Zellen und Verfahren zur Unterdrückung einer Immunantwort offenbart. Bei diesen Verfahren werden Blutzellen oder T-Zellen mit einer Menge an TGF-β oder einer zu TGF-β analogen Verbindung und einem Agonisten für den Retinolsäure-Rezeptor, oder mit einer Menge eines Retinoid-X-Rezeptors oder eines PPARγ-Agonisten in Kontakt gebracht, die ausreicht, die Differenzierung zu regulatorischen T-Zellen zu stimulieren.

Die vorliegende Erfindung basiert auf folgenden experimentellen Ergebnissen:
Die Aktivierung von PPARγ in CD4⁺ T-Zellen während der Differenzierung zu T_{H}17-Zellen ("T_{H}17-Differenzierung") führt zu einer ausgeprägten Inhibition der IL-17A Expression. Dieser Effekt war nach Aktivierung von PPARγ sowohl durch synthetische PPARγ-Agonisten Pioglitazon und Rosiglitazon als auch durch die endogenen PPARγ-Agonisten 9s-HODE und 13s-HODE nachweisbar. Da der Effekt in PPARγ knock-out T-Zellen nicht nachweisbar war, handelt es sich um einen PPARγ-spezifischen Effekt. Der PPARγ-vermittelte supprimierende Effekt war nicht nur beschränkt auf die Produktion von IL-17A, sondern auch auf die Expression von IL-17F, TNFα, IL-22 und IL-23R nachweisbar.

Da nur eine Suppression der T_{H}17-Differenzierung, jedoch nicht der T_{H}1-Differenzierung und der T_{H}2-Differenzierung nachweisbar war, handelt es sich bei diesem PPARγ-vermittelten Effekt um eine selektive Suppression.

Eine PPARγ-Aktivierung hemmte die IL-17A-Produktion durch CD4⁺ T-Zellen auch während antigen-spezifischer T-Zell-Aktivierung. Aktivierung von PPARγ in CD4⁺ T-Zellen während der T_{H}17-Differenzierung resultierte in einer signifikanten Hemmung der Expression des Transkriptionsfaktors RORγt, der eine entscheidende Rolle in der Induktion der T_{H}17-Differenzierung spielt. Darüber hinaus konnten wir zeigen, dass die Aktivierung von PPARγ zu einer Protektion vor entzündlich bedingtem Abbau des Corepressormoleküls SMRT in T-Zellen führt. Dieses Molekül bindet unter nichtentzündlichen Konditionen auf dem RORγt-Promoter und verhindert somit dessen Aktivierung und dadurch die Expression von RORγt. Nach Aktivierung der Zellen wird SMRT vom RORγt Promoter entfernt und somit die Expression von RORγt ermöglicht.

In-vivo führte die orale Behandlung von C57/B16 Mäusen mit dem PPARγ-Agonisten Pioglitazone nicht nur zu einer deutlichen Reduktion der klinischen Krankheitssymptome, sondern reduzierte signifikant die Produktion von IL-17A sowohl peripher als auch im Zielorgan, also dem zentralen Nervensystem. In Mäusen mit einem T-Zell-spezifischen knock-out von PPARγ wurde im Verlauf der EAE eine gesteigerte IL-17A-Produktion peripher und im zentralen Nervensystem beobachtet. In diesen konditionalen PPARγ knock-out Mäusen war die Pioglitazon-mediierte Reduktion der klinischen Krankheitsmanifestation und die Suppression der IL-17A Produktion durch CD4⁺ T Zellen nicht mehr nachweisbar.

Zusätzlich konnte eine selektive Suppression der T_{H}17-Differenzierung, jedoch nicht der T_{H}1-Differenzierung durch PPARγ-Agonisten in humanen CD4+ T-Zellen von gesunden Probanden und MS Patienten nachgewiesen werden.

Diese Ergebnisse zeigen, dass die Reduktion der T_{H}17-Differenzierung von murinen und humanen CD4+ T-Zellen und der protektive Effekt im Tiermodell der Multiplen Sklerose durch pharmakologische PPARγ-Aktivierung in CD4⁺ T-Zellen vermittelt werden.

Durch diese Ergebnisse eröffnet sich die Möglichkeit, Erkrankungen zu behandeln, bei denen T_{H}17-Zellen eine pathophysiologisch relevante Funktion haben, indem PPARγ pharmakologisch aktiviert wird.

Die vorliegende Erfindung betrifft somit Verfahren zur Behandlung von Erkrankungen, bei denen T_{H}17-Zellen eine pathophysiologisch relevante Funktion haben, insbesondere Erkrankungen, bei denen die Hemmung der T_{H}17-Differenzierung und/oder der Aktivität von T_{H}17-Zellen ein therapeutisches Ziel darstellt. Letzteres kann beispielsweise die Hemmung der Aktivität und/oder die Reduktion der Expression des Transkriptionsfaktors RORγt sein.

Zu den Erkrankungen, bei denen T_{H}17-Zellen eine pathophysiologisch relevante Funktion haben, gehören beispielsweise rheumatoide Arthritis, Psoriasis, Multiple Sklerose, chronisch entzündliche Darmerkrankungen wie Morbus Crohn und Colitis ulcerosa, systemischer Lupus erythematodes, cerebrovaskulärer Insult, allergisches Asthma bronchiale sowie akutes Koronarsyndrom.

In einer besonderen Ausführungsform umfasst das erfindungsgemäße Verfahren zur Behandlung von Erkrankungen, bei denen T_{H}17-Zellen eine pathophysiologisch relevante Funktion haben, die Behandlung von Patienten, die resistent gegenüber anderen immunmodulatorischen oder immunsuppressiven Arzneimitteln sind, mit PPARγ-Agonisten.

Das erfindungsgemäße Verfahren zur Behandlung von Erkrankungen, bei denen T_{H}17-Zellen eine pathophysiologisch relevante Funktion haben, umfasst die Verabreichung mindestens eines PPARγ-Agonisten. Vorzugsweise werden bei der Behandlung dieser Erkrankungen ausschließlich ein oder mehrere PPARγ-Agonisten verabreicht. Es ist jedoch auch möglich, die Verabreichung eines oder mehrerer PPARγ-Agonisten mit anderen pharmazeutischen Wirkstoffen zu kombinieren, die zur Behandlung einer von T_{H}17-Zellen vermittelten Erkrankung eingesetzt werden können.

Die vorliegende Erfindung umfasst somit auch die Verwendung von PPARγ-Agonisten zur Behandlung von Erkrankungen, bei denen T_{H}17-Zellen eine pathophysiologisch relevante Funktion haben, sowie die Verwendung von PPARγ-Agonisten zur Herstellung eines Medikaments dieser Erkrankungen. Daher erstreckt sich die vorliegende Erfindung auch auf Medikamente zur Behandlung von Erkrankungen, bei denen T_{H}17-Zellen eine pathophysiologisch relevante Funktion haben, wobei sich die Medikamente dadurch auszeichnen, dass sie mindestens einen PPARγ-Agonisten enthalten.

In einem weiteren Aspekt umfasst die vorliegende Erfindung auch die Verwendung von PPARγ-Agonisten zur Reduktion der Differenzierung von CD4⁺ T-Lymphozyten zu T_{H}17-Lymphozyten und/oder zur Hemmung der Aktivität und/oder Reduktion der Expression des Transkriptionsfaktors RORγt. Die vorliegende Erfindung betrifft ferner ein Verfahren zur Reduktion der Differenzierung von CD4⁺ T-Lymphozyten zu T_{H}17-Lymphozyten und/oder zur Hemmung der Aktivität und/oder Reduktion der Expression des Transkriptionsfaktors RORγt, wobei mindestens ein PPARγ-Agonist verwendet wird.

Zur Behandlung der Erkrankungen, bei denen T_{H}17-Zellen eine pathophysiologisch relevante Funktion haben, können der PPARγ-Agonist oder die PPARγ-Agonisten in oraler Form oder in parenteraler Form verabreicht werden. Medikamente liegen vorzugsweise als oral zu verabreichende Darreichungsform oder als parenteral zu verabreiche Darreichungsform vor. Geeignete orale Darreichungsformen sind zum Beispiel, ohne Einschränkung, Tabletten, die gegebenenfalls mit einer magensaftresistenten Beschichtung versehen sind, Kapseln, Pillen, Granulate, Elixiere, Lösungen, Suspensionen, Sirup, feste oder flüssige Aerosole. Parenterale Verabreichung schließt Darreichungsformen für die subkutane, intravenöse, intraarterielle und intramuskuläre Verabreichung der PPARγ-Agonisten ein. Außerdem können PPARγ-Agonisten über Inhalation oder in anderen Formulierungen über den Respirationstrakt (zum Beispiel intranasal) verabreicht werden.

Zur Behandlung der Erkrankungen, bei denen T_{H}17-Zellen eine pathophysiologisch relevante Funktion haben, können unterschiedliche Dosierungen, auch in Abhängigkeit der Darreichungsform, oder Arzneimittel mit einer zur Behandlung der jeweiligen Erkrankung wirksamen Dosis an mindestens einem PPARγ-Agonisten zur Anwendung kommen.

Zur Behandlung der Erkrankungen, bei denen T_{H}17-Zellen eine pathophysiologisch relevante Funktion haben, können die PPARγ-Agonisten nach verschiedenen Verabreichungsschemata verabreicht werden, die von etwa von 3x täglich bis 1x wöchentlich reichen. Die PPARγ-Agonisten können auch bei Verdacht auf eine T_{H}17-vermittelte Autoimmunerkrankung oder zur Therapie bei nachgewiesener T_{H}17-vermittelter Autoimmunerkrankung eingesetzt werden.

Bevorzugte PPARγ-Agonisten zur Behandlung von Erkrankungen, bei denen T_{H}17-Lymphozyten eine pathophysiologisch relevante Funktion haben, oder zur Herstellung von Medikamenten zur Behandlung dieser Erkrankungen sind aus der Gruppe von pharmazeutischen Wirkstoffen ausgewählt, die Farglitazar, Aleglitazar, Muraglitazar und Thiazolidindione, beispielsweise Pioglitazon, Ciglitazon, Troglitazon, Rivoglitazon und Rosiglitazon, umfasst. Als weitere Verbindungen, die PPARγ-Agonisten sind, können beispielsweise MCC-555 (CAS-Nr. 161600-01-7), ein strukturelles Homolg des Rosiglitazons und anderer Thiazolidindione und 15-Deoxy-A-12,14-prostagladin J₂ (15dPGJ₂) genannt werden.

Die nachfolgenden Beispiele dienen der Veranschaulichung der vorliegenden Erfindung, ohne diese in irgendeiner Weise einzuschränken.

### Beispiel 1

In-vitro Behandlung von CD4⁺ T-Lymphozyten mit PPARγ-Agonisten während der T_{H}17-Differenzierung
Naive CD4⁺ T-Lymphozyten wurden aus Milzen von C57/B16 Mäusen oder von CD4-Cre PPARγ^{fl/fl} Mäusen (Mäuse mit einem T-Zell-spezifischen knock-out von PPARγ; die Mäuse wurden durch Verkreuzung von CD4-Cre Mäusen mit PPARγ^{fl/fl} Mäusen generiert) mittels magnetischer Zellseparation (CD4-beads, Miltenyi) isoliert. Die Zellen wurden unter Standardbedingungen kultiviert (RPMI, 8% FCS, 2 mM Glutamin, 100 U/ml Penicillin, 100 µg/ml Streptomycin bei 37°C in 5% CO₂ in einem Feuchtinkubator). Die Zellen wurden in einer Dichte von ca. 150.000 Zellen/Well in insgesamt 300 µl oben genannten Mediums/Well in einer 48-Well Platte ausgesät. Zur Aktivierung der Zellen wurde die Platte vorher mit anti (α)CD3 und αCD28 Antikörpern beschichtet, jeweils in einer Konzentration von 4 µg/ml in PBS für 1h, anschließend wurde die Platte dreimal mit PBS gewaschen. Zur Induktion der T_{H}17-Differenzierung wurden die Zytokine TGFβ (5 ng/ml) und IL-6 (20 ng/ml) während des gesamten Assays zugegeben. Zusätzlich wurden neutralisierende Antikörper gegen IL-4 und gegen IFNγ in einer Konzentration von jeweils 10 µg/ml zugegeben. Folgende PPARγ-Agonisten wurden während des gesamten Assays zugegeben: Pioglitazone (10 µM; Stocklösung 10 mM in DMSO; Axxora); 13s-HODE (10 µM, 100 µg/ml Stammlösung in Ethanol; Cayman Chemicals). 72h Stunden nach Versuchsbeginn wurden die CD4⁺ T-Zellen für 4-5h restimuliert mit PMA (5 ng/ml) und lonomycin (200 ng/ml) in Anwesenheit von Monensin und Brefeldin A (jeweils von eBioscience) und anschließend der Prozentsatz der IL-17A produzierenden T-Zellen mittels intrazellulärer Färbung mit einem APC-markierten Antikörper gegen IL-17A und anschließender durchflußzytometrischer Analyse der Zellen quantifiziert. Die Färbung erfolgte gemäß den Angaben des Herstellers. Die Analyse erfolgte an einem Canto II (BD Biosciences); die Auswertung mit Hilfe der Software FlowJo (TreeStar). Für die Analyse der IL-17A Produktion von CD4⁺ T-Zellen nach antigenspezifischer Aktivierung wurden ovalbuminspezifische CD4⁺ T-Zellen aus Milzen von OT-II Mäusen mittels immunomagnetischer Zellseparation isoliert und kokultiviert mit CD11c⁺ dendritischen Zellen aus Milzen von C57/BI6 Mäusen in Anwesenheit des MHC Klasse II Peptids ova₃₂₃₋₃₃₉. Die Zytokine TGFβ und IL-6, sowie die neutralisierenden Antikörper gegen IL-4 und gegen IFNγ wurden zugegeben wie oben beschrieben. Nach 72h wurden die Zellen wie oben beschrieben mit PMA und lonomycin restimuliert und die IL-17A Produktion durchflußzytometrisch quantifiziert. Zur T_{H}1-Induktion wurden die CD4⁺ T-Zellen mit gecoateten αCD3 und αCD28 Antikörpern (je 4µg/ml), IL-12 (10ng/ml) in Anwesenheit von αIL-4 differenziert. Die Färbung erfolgte analog mit einem PE-markierten Antikörper gegen αIFNγ. Die Expression des Transkriptionsfaktors RORγt wurde in isolierten CD4⁺ T-Zellen 16h nach Beginn der T_{H}17-Differenzierung mittels real-time PCR analysiert. Die Differenzierung erfolgte wie oben beschrieben; nach 16h wurden die Zellen mit PBS gewaschen und anschließend die RNA mit Hilfe des Qiagen RNeasy Kits extrahiert. Die RNA wurde mit dem Supercript III Kit von Invitrogen in cDNA umgeschrieben. Die Primersequenzen für die Detektion von RORγt sind 5'-tcagtcatgagaacacaaattgaa und 5'-ggtgataaccccgtagtgga. Die Genexpression wurde normalisiert im Verhältnis zu β-Aktin. Die Analyse wurde mit einem AbiPrism 7900 HT cycler durchgeführt.

Die Aktivierung von PPARγ mit dem Agonisten Pioglitazon (PIO) während der Differenzierung von naiven CD4⁺ T-Zellen zu T_{H}17-Effektor-T-Zellen führte zu einer signifikanten Reduktion der IL-17A Produktion, gemessen nach Restimulation der Zellen 72h nach Beginn der Differenzierung mit anti-CD3 (4 µg/ml), anti-CD28 (4 µg/ml), TGFβ (5 ng/ml) und IL-6 (20 ng/ml). Knock-out von PPARγ steigerte hingegen die T_{H}17-Induktion. Die Differenzierung von IFNγ-produzierenden T_{H}1-Zellen wurde hingegen nicht durch PPARγ beeinflusst.

Der supprimierende Effekt der PPARγ-Aktivierung mit Pioglitazon war auch nach antigenspezifischer CD4⁺ T-Zellaktivierung in Anwesenheit der T_{H}17-induzierenden Zytokine TGFβ und IL-6 nachweisbar. Ovalbuminspezifische CD4⁺ T-Zellen aus TCR-transgenen OTII Mäusen wurden durch Ovalbumin-Peptid-beladene dendritische Zellen für 72h aktiviert und anschließend nach 4h Restimulation mit PMA und lonomycin der Prozentsatz der IL-17A produzierenden CD4⁺ T-Zellen durchflusszytometrisch bestimmt, wobei eine Suppression der TGFβ-vermittelten Induktion FoxP3 positiver regulatorischer T-Zellen durch pharmakologische PPARγ Aktivierung jedoch nicht nachweisbar war.

Knock-out von PPARγ in CD4⁺ T-Zellen führte zu einer gesteigerten Expression des Transkriptionsfaktors RORγt unter T_{H}17 induzierenden Bedingungen, während Behandlung mit PIO die RORγt Expression supprimiert. Induktion der T_{H}17-Differenzierung von naiven CD4⁺ T-Zellen aus Wildtyp beziehungsweise CD4-PPARγ^{KO} Mäusen mit αCD3 (4 µg/ml), αCD28 (4 µg/ml), TGFβ (5 ng/ml) und IL-6 (20 ng/ml) für 16h. Nach Extraktion der RNA wurde die T_{H}17-abhängige RORγt Expression mittels real-time PCR quantifiziert und β-actin diente als interne Kontrolle.

Ligandenaktiviertes PPARγ stabilisierte den nukleären Korepressor SMRT auf dem RORγt-Promoter und reduzierte hierdurch dessen TGFβ/IL-6 induzierte Expression, nachgewiesen indem murine CD4+ T-Zellen mit αCD3 (4 µg/ml), αCD28 (4 µg/ml), TGFβ (5 ng/ml) und IL-6 (20 ng/ml) in Anwesenheit bzw. Abwesenheit von Pioglitazon (PIO) stimuliert, der nukleäre Korepressor SMRT anschließend immunpräzipitiert und die Menge des im Präzipitat nachweisbaren RORγt Promoters mittels quantitativer PCR analysiert wurde.

### Beispiel 2

In-vivo Behandlung von Mäusen mit dem PPARγ-Agonisten Pioglitazon
7 Tage vor Beginn der Immunisierung bis zum Ende des Experiments wurden die Mäuse mit jeweils 30 mg/kg Körpergewicht pro Tag Pioglitazon (Actos^{™}, Takeda Pharma, Aachen, Germany) suspendiert in 0,5% Carboxymethylcellulose per os mit Hilfe einer Schlundsonde gefüttert. Kontrollmäuse wurden mit 0,5% Carboxymethylcellulose gefüttert.

Die experimentelle autoimmune Encephalomyelitis (EAE) wurde in 8- bis 12 Wochen alten C57/BI6 Mäusen oder in CD4-PPARγ^{KO} Mäusen und ihren Cre-negativen Wildtyp-Geschwistertieren durch subkutane Injektion von 50 µg MOG₃₅₋₅₅ Peptid (Biotrend, Cologne, Germany) und 8 mg/ml Hitze-inaktiviertem *Mycobacterium tuberculosis* (Stamm H37RA; Difco Laboratories, Detroit, USA) in Complete Freund's Adjuvant (Difco Laboratories) am Tag 0 in die Schwanzwurzel induziert. Zusätzlich wurden zwei Injektionen *Bordetella pertussis toxin* (List Biologicals, Campbell, USA; 200 ng pro Maus und Injektion) intraperitoneal jeweils an Tag 0 und an Tag 2 appliziert. Die Tiere wurden täglich klinisch untersucht. Die Krankheitsaktivität wurde anhand einer Skala von 0 bis 6 quantifiziert: 0 = klinisch unauffällig; 1 = reduzierter Schwanztonus; 2 = Ataxie und/oder leichte Hinterbeinparese; 3 = schwere Parese der Hinterbeine; 4 = Plegie der Hinterbeine; 5 = Tetraparese; 6 = Tod.

Bei einigen Versuchen wurden die Tiere zum Zeitpunkt des Erkrankungsmaximums (d15) getötet, Gehirn und Rückenmark isoliert, das Gewebe mechanisch zerkleinert und mit 1 mg/ml Collagenase la (Sigma) in PBS für 45 min bei 37°C verdaut. Anschließend wurden die Immunzellen mit Hilfe eines Percoll-Gradienten (30%/37%/70%) aufgereinigt. CD4⁺ T-Zellen aus den Milzen der Tiere wurden mittels immunomagnetischer Zellseparation aufgereinigt. Die Zellen wurden in oben angegebenem Medium zusammen mit aufgereinigten CD11c⁺ dendritischen Zellen in Anwesenheit von MOG₃₅₋₅₅ (10 µg/ml) auf anti-IL-17A-Antikörper-beschichteten 96-well Platten für 24h kokultiviert. Anschließend wurden die Zellen abgewaschen und die Platten entsprechend dem Herstellerprotokoll entwickelt (EL421; R&D Systems, Wiesbaden, Germany). Die Anzahl positiver Spots wurde mit Hilfe eines automatischen ELISpot reader quantifiziert (BIOREADER-2000, Biosys, Karben, Germany).

Orale Behandlung von Mäusen mit Pioglitazon reduzierte die klinische Symptomatik sowie die antigenspezifische T_{H}17-Antwort im zentralen Nervensystem (ZNS) während Experimenteller Autoimmuner Enzephalomyelitis (EAE). Wildtyp Mäuse wurden ab Tag -7 bis zum Ende des jeweiligen Experimentes mit Pioglitazon oder der Trägersubstanz Carboxymethylcellulose täglich behandelt und ab Tag 0 die MOG-EAE induziert. Die Krankheitssymptome waren in der mit Pioglitazon behandelten Gruppe kaum ausgeprägt, während sie bei der unbehandelten Kontrollgruppe einen Wert von 2 auf der Skala erreichten.

Bei am Tag 15 (Erkrankungsmaximum) aus dem zentralen Nervensystem isolierten und mit MOG-Peptid-beladenen dendritischen Zellen restimulierten CD4+ T-Zellen war die Anzahl IL-17A positiver Spots im Elispot nach 24h Restimulation der T-Zellen aus dem ZNS bei den mit Pioglitazon behandelten Mäusen signifikant niedriger als bei den nicht behandelten Mäusen.

CD4-Cre PPARγ^{KO} Mäuse wiesen zudem einen früheren Erkrankungsbeginn und einen während der intitialen T-Zell-abhängigen Phase schwereren Krankheitsverlauf auf, wie durch tägliches dokumentieren der Krankheitsverläufe bei CD4-Cre PPARγ^{KO} Mäusen und ihren Cre-negativen Wildtyp-Geschwistertieren, bei denen eine MOG-EAE induziert wurde, gezeigt werden konnte. und anschließend der Krankheitsverlauf täglich dokumentiert.

Bei am Tag 15 aus dem ZNS isolierten und mit MOG-Peptid-beladenen dendritischen Zellen restimulierten CD4⁺ T-Zellen war die Anzahl IL-17A positiver Spots im Elispot nach 24h Restimulation der T-Zellen aus dem ZNS von CD4-Cre PPARγ^{KO} Mäusen deutlich höher als die Anzahl der IL-17A positiven Spots der T-Zellen aus dem ZNS der Cre-negativen Wildtyp-Geschwistertiere.

### Beispiel 3

### Chromatin-Immunpräzipitation

In CD4⁺ T-Zellen von C57/B16 Mäusen wurde in Anwesenheit bzw. Abwesenheit des PPARγ-Agonisten Pioglitazon (PIO 10 µM)) die T_{H}17-Differenzierung induziert wie oben beschrieben. Zu verschiedenen Zeitpunkten wurden die Zellen für 10 min mit 1 % Formaldehyd behandelt, um die Protein-DNA-Interaktionen zu stabilisieren. Die Proben wurden entweder mit einem SMRT-spezifischen Antikörper (von ABR) oder einem Kontroll Aktikörper (rabbit-IgG) präzipitiert. Anschließend wurde eine 150bp Region des RORγt-Promoters aus dem Präzipitat mittels SYBR-green-basierter quantitativer real-time PCR amplifiziert und mit Hilfe eines 7200 PCR systems (ABI) analysiert.

### Beispiel 4

T_{H}17-Differenzierung von humanen CD4+ T-Zellen von MS Patienten

CD4⁺ T Lymphozyten wurden aus PBMCs (peripheren mononukleären Zellen aus dem Blut) von MS Patienten mittels magnetischer Zellseparation (naive CD4-T cell isolation kit, Miltenyi) isoliert. Die Zellen wurden kultiviert in X-VIVO Medium (mit 100 U/ml Penicillin und 100 µg/ml Streptomycin) bei 37°C in 5% CO₂ in einem Feuchtinkubator. Die Zellen wurden in einer Dichte von ca. 150.000 Zellen/Well in insgesamt 300µl oben genannten Mediums/Well in einer 48-Well Platte ausgesät. Zur Aktivierung der Zellen wurde die Platte vorher mit anti-humanen αCD3- und αCD28-Antikörpern beschichtet, jeweils in einer Konzentration von 1,5 µg/ml in PBS für 1h. Anschließend wurde die Platte dreimal mit PBS gewaschen. Zur Induktion der T_{H}17-Differenzierung wurden die Zytokine TGFβ (5 ng/ml) und IL-21 (20 ng/ml) während des gesamten Assays zugegeben. Der PPARγ-Agonist Pioglitazon (10 µM; Stammlösung 10 mM in DMSO; Axxora) wurde während der gesamten Differenzierungsphase zugegeben. 7 Tage nach Versuchsbeginn wurden die Überstände hinsichtlich der Konzentration von IL-17A und IFNγ mittels ELISA untersucht; hierfür wurden kommerziell erhältliche Kits von R&D systems verwendet. Alternativ wurde die Expression der T_{H}17-Markermoleküle IL-17F, IL-21, IL-22 und IL-23R mittels real-time PCR unter Verwendung kommerziell erhältlicher Sonden (ABI) analysiert. Zur Analyse der Expression der Trankriptionsfaktoren RORγt, T-bet und GATA-3 wurden isolierte humane CD4+ T-Zellen differenziert wie oben beschrieben und die Expression der Transkriptionsfaktoren nach 48h mittels real-time PCR unter Verwendung kommerziell erhältlicher Sonden analysiert.

Die Ergebnisse zeigen, dass Pioglitazon die T_{H}17-Differenzierung humaner CD4+ T-Zellen von MS Patienten supprimiert, nicht jedoch die T_{H}1-Differenzierung von humanen CD4+ T-Zellen. Gemessen wurden die Produktion von IL-17A beziehungsweise von IFNγ nach Stimulation isolierter CD4⁺ T-Zellen von MS Patienten mit αCD3 und αCD28 in Anwesenheit von TGFβ und IL-21 nach 7 Tagen. Analog zur Suppression von IL-17A wurde auch die Expression weiterer Marker von T_{H}17-Zellen, nämlich IL-23R, IL-22, IL-21 und IL-17F, durch Pioglitazon supprimiert. Pioglitazon supprimiert die Expression des Transkriptionsfaktors RORγt, nicht jedoch die Expression von Tbet und GATA-3 in humanen CD4⁺ T Zellen von MS Patienten.

## Patentansprüche

1. Verwendung eines oder mehrerer PPARγ-Agonisten zur Herstellung eines Medikaments zur Behandlung einer Erkrankung, bei denen T_{H}17-Zellen eine pathophysiologisch relevante Funktion haben.

2. Verwendung nach Anspruch 1, **dadurch gekennzeichnet, dass** die Erkrankung, bei denen T_{H}17-Zellen eine pathophysiologisch relevante Funktion haben, aus der Gruppe von Erkrankungen ausgewählt ist, die rheumatoide Arthritis, Psoriasis, Multiple Sklerose, chronisch entzündliche Darmerkrankungen wie Morbus Crohn und Colitis ulcerosa, systemischen Lupus erythematodes, cerebrovaskulären Insult, allergisches Asthma bronchiale sowie akutes Koronarsyndrom umfasst.

3. Verwendung nach Anspruch 1 oder 2, **dadurch gekennzeichnet, dass** der PPARγ-Agonist oder mindestens einer der PPARγ-Agonisten aus der Gruppe ausgewählt ist, die Farglitazar, Aleglitazar, Muraglitazar und Thiazolidindione umfasst.

4. Verwendung nach Anspruch 3, **dadurch gekennzeichnet, dass** die Thiazolidindione aus der Gruppe ausgewählt sind, die Pioglitazon, Ciglitazon, Troglitazon, Rivoglitazon und Rosiglitazon umfasst.

5. Verwendung nach einem der vorhergehenden Ansprüche, **dadurch gekennzeichnet, dass** das Medikament als oral zu verabreichende Darreichungsform oder als parenteral zu verabreiche Darreichungsform vorliegt.

6. Medikament zur Behandlung einer Erkrankung, bei der T_{H}17-Zellen eine pathophysiologisch relevante Funktion haben, enthaltend eine therapeutisch wirksame Menge mindestens eines PPARγ-Agonisten.

7. Medikament nach Anspruch 6, **dadurch gekennzeichnet, dass** die Erkrankung, bei denen T_{H}17-Zellen eine pathophysiologisch relevante Funktion haben, aus der Gruppe von Erkrankungen ausgewählt ist, die rheumatoide Arthritis, Psoriasis, Multiple Sklerose, chronisch entzündliche Darmerkrankungen wie Morbus Crohn und Colitis ulcerosa, systemischen Lupus erythematodes, cerebrovaskulären Insult, allergisches Asthma bronchiale sowie akutes Koronarsyndrom umfasst.

8. Medikament nach Anspruch 6 oder 7, **dadurch gekennzeichnet, dass** der PPARγ-Agonist oder mindestens einer der PPARγ-Agonisten aus der Gruppe ausgewählt ist, die Farglitazar, Aleglitazar, Muraglitazar und Thiazolidindione umfasst.

9. Medikament nach einem der Ansprüche 6 bis 8, **dadurch gekennzeichnet, dass** die Thiazolidindione aus der Gruppe ausgewählt sind, die Pioglitazon, Ciglitazon, Troglitazon, Rivoglitazon und Rosiglitazon umfasst.

10. Medikament nach einem der Ansprüche 6 bis 9, **dadurch gekennzeichnet, dass** es als oral zu verabreichende Darreichungsform oder als parenteral zu verabreiche Darreichungsform vorliegt.

11. Verfahren zur Reduktion der Differenzierung von CD4+ T-Lymphozyten zu T_{H}17-Lymphozyten und/oder zur Hemmung der Aktivität und/oder Reduktion der Expression des Transkriptionsfaktors RORγt, **dadurch gekennzeichnet, dass** mindestens ein PPARγ-Agonist verwendet wird.

12. Verfahren nach Anspruch 11, **dadurch gekennzeichnet, dass** der PPARγ-Agonist oder mindestens einer der PPARγ-Agonisten aus der Gruppe ausgewählt wird, die Farglitazar, Aleglitazar, Muraglitazar, Pioglitazon, Ciglitazon, Troglitazon, Rivoglitazon, Rosiglitazon, MCC-555 und 15dPGJ₂ umfasst.

13. Verfahren zur Behandlung einer Erkrankung, bei der T_{H}17-Zellen eine pathophysiologisch relevante Funktion haben, **gekennzeichnet durch** die Verabreichung mindestens eines PPARγ-Agonisten in therapeutisch wirksamer Menge.
